# EUROPEAN PATENT APPLICATION

(11) **EP 3 023 791 A1**
(43) Date of publication of application: **25.05.2016**
(21) Application number: 14194296.1
(22) Date of filing: 21.11.2014
(51) Int. Cl.: G01N 33/574

(54) **Predicting the responsiveness to gemcitabine treatment**

(71) Applicant: Atlas Antibodies AB, 106 91 Stockholm (SE)
(72) Inventor: Jirström, Karin, 216 18 Limhamn (SE); Eberhard, Jakob, 224 77 Lund (SE)
(74) Representative: Kransell & Wennborg KB

(57) **Abstract**

There is provided a method of determining whether a mammalian subject having a cancer belongs to a first or a second group, wherein subjects of the first group are more responsive to gemcitabine than subjects of the second group, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,

if said sample value is higher than said reference value,
c1) concluding that said subject belongs to said first group; and
if said sample value is lower than or equal to said reference value,
c2) concluding that said subject belongs to said second group.

SEQUENCE LISTING

<110>  Atlas Antibodies AB

<120>  PREDICTING THE RESPONSIVENESS TO GEMCITABINE TREATMENT

<130>  14765EP

<160>  28

<170>  PatentIn version 3.5

<210>  1
<211>  134
<212>  PRT
<213>  Homo sapiens

<400>  1

Asp Glu Gln Ala Leu Glu Asp His Phe Ser Ser Phe Gly Pro Ile Ser
1               5                   10                  15

Glu Val Val Val Val Lys Asp Arg Glu Thr Gln Arg Ser Arg Gly Phe
            20                  25                  30

Gly Phe Ile Thr Phe Thr Asn Pro Glu His Ala Ser Val Ala Met Arg
        35                  40                  45

Ala Met Asn Gly Glu Ser Leu Asp Gly Arg Gln Ile Arg Val Asp His
    50                  55                  60

Ala Gly Lys Ser Ala Arg Gly Thr Arg Gly Gly Gly Phe Gly Ala His
65                  70                  75                  80

Gly Arg Gly Arg Ser Tyr Ser Arg Gly Gly Gly Asp Gln Gly Tyr Gly
                85                  90                  95

Ser Gly Arg Tyr Tyr Asp Ser Arg Pro Gly Gly Tyr Gly Tyr Gly Tyr
            100                 105                 110

Gly Arg Ser Arg Asp Tyr Asn Gly Arg Asn Gln Gly Gly Tyr Asp Arg
        115                 120                 125

Tyr Ser Gly Gly Asn Tyr
    130

<210>  2
<211>  157
<212>  PRT
<213>  Homo sapiens

<400>  2

Met Ser Ser Glu Glu Gly Lys Leu Phe Val Gly Gly Leu Asn Phe Asn

1               5                   10                  15

Thr Asp Glu Gln Ala Leu Glu Asp His Phe Ser Ser Phe Gly Pro Ile
            20                  25                  30

Ser Glu Val Val Val Val Lys Asp Arg Glu Thr Gln Arg Ser Arg Gly
        35                  40                  45

Phe Gly Phe Ile Thr Phe Thr Asn Pro Glu His Ala Ser Val Ala Met
    50                  55                  60

Arg Ala Met Asn Gly Glu Ser Leu Asp Gly Arg Gln Ile Arg Val Asp
65                  70                  75                  80

His Ala Gly Lys Ser Ala Arg Gly Thr Arg Gly Gly Gly Phe Gly Ala
                85                  90                  95

His Gly Arg Gly Arg Ser Tyr Ser Arg Gly Gly Gly Asp Gln Gly Tyr
            100                 105                 110

Gly Ser Gly Arg Tyr Tyr Asp Ser Arg Pro Gly Gly Tyr Gly Tyr Gly
        115                 120                 125

Tyr Gly Arg Ser Arg Asp Tyr Asn Gly Arg Asn Gln Gly Gly Tyr Asp
    130                 135                 140

Arg Tyr Ser Gly Gly Asn Tyr Arg Asp Asn Tyr Asp Asn
145                 150                 155

<210>  3
<211>  1130
<212>  DNA
<213>  Homo sapiens

<400>  3
atgcgcaatg tggcccccta atggtggctg cgctgagcca gctcctcaga ttaccacctt      60

attggccgcc tttctcagct tttctgtagt tacccatatt ttgttcctct ttcttgtcta     120

ttttctgtgc tttttctctg ctttccgtct cgctattttc tcacatctcc attttctttc      180

tccttcctgc caccattctt catgttcttc ccacaggact tgaactgcca tgtcctctga     240

agaaggaaag ctcttcgtgg gagggctcaa ctttaacacc gacgagcagg cactggaaga     300

ccacttcagc agtttcggac ctatctctga ggtggtcgtt gtcaaggacc gggagactca     360

gcggtccagg ggttttggtt tcatcacctt caccaaccca gagcatgctt cagttgccat     420

gagagccatg aacggagagt ctctggatgg tcgtcagatc cgtgtggatc atgcaggcaa     480

gtctgctcgg ggaaccagag gaggtggctt tggggcccat gggcgtggtc gcagctactc     540

tagaggtggt ggggaccagg gctatgggag tggcaggtat tatgacagtc gacctggagg 600

gtatggatat ggatatggac gttccagaga ctataatggc agaaaccagg gtggttatga 660

ccgctactca ggaggaaatt acagagacaa ttatgacaac tgaaatgaga catgcacata 720

atatagatac acaaggaata atttctgatc caggatcgtc cttccaaatg gctgtattta 780

taaaggtttt tggagctgca ccgaagcatc ttattttata gtatatcaac cttttgtttt 840

taaattgacc tgccaaggta gctgaagacc ttttagacag ttccatcttt ttttttaaat 900

tttttctgcc tatttaaaga caaattatgg gacgtttgta gaacctgagt atttttcttt 960

ttaccagttt tttagtttga gctcttaggt ttattggagc tagcaataat tggttctggc 1020

aagtttggcc agactgactt caaaaaatta atgtgtatcc agggacattt taaaaacctg 1080

tacacagtgt ttattgtggt taggaagcaa tttcccaatg tacctataag 1130

<210> 4
<211> 29
<212> PRT
<213> Homo sapiens

<400> 4

Arg Gly Phe Gly Phe Ile Thr Phe Thr Asn Pro Glu His Ala Ser Val
1               5                   10                  15

Ala Met Arg Ala Met Asn Gly Glu Ser Leu Asp Gly Arg
            20                  25

<210> 5
<211> 24
<212> PRT
<213> Homo sapiens

<400> 5

Arg Ser Tyr Ser Arg Gly Gly Gly Asp Gln Gly Tyr Gly Ser Gly Arg
1               5                   10                  15

Tyr Tyr Asp Ser Arg Pro Gly Gly
            20

<210> 6
<211> 5
<212> PRT
<213> Homo sapiens

<400> 6

Thr Phe Thr Asn Pro
1               5

<210> 7

<211>  10
<212>  PRT
<213>  Homo sapiens

<400>  7

Gly Thr Arg Gly Gly Gly Phe Gly Ala His
1               5                   10


<210>  8
<211>  10
<212>  PRT
<213>  Homo sapiens

<400>  8

Gly Phe Gly Ala His Gly Arg Gly Arg Ser
1               5                   10


<210>  9
<211>  5
<212>  PRT
<213>  Homo sapiens

<400>  9

Tyr Asp Arg Tyr Ser
1               5


<210>  10
<211>  9
<212>  PRT
<213>  Homo sapiens

<400>  10

Asp Glu Gln Ala Leu Glu Asp His Phe
1               5


<210>  11
<211>  7
<212>  PRT
<213>  Homo sapiens

<400>  11

Thr Asn Pro Glu His Ala Ser
1               5


<210>  12
<211>  10
<212>  PRT
<213>  Homo sapiens

<400>  12

Glu His Ala Ser Val Ala Met Arg Ala Met
1               5                   10

<210>  13
<211>  10
<212>  PRT
<213>  Homo sapiens

<400>  13

Arg Gly Gly Gly Phe Gly Ala His Gly Arg
1               5                   10


<210>  14
<211>  8
<212>  PRT
<213>  Homo sapiens

<400>  14

Gly Phe Gly Ala His Gly Arg Gly
1               5


<210>  15
<211>  9
<212>  PRT
<213>  Homo sapiens

<400>  15

Asp Tyr Asn Gly Arg Asn Gln Gly Gly
1               5


<210>  16
<211>  10
<212>  PRT
<213>  Homo sapiens

<400>  16

His Gly Arg Gly Arg Ser Tyr Ser Arg Gly
1               5                   10


<210>  17
<211>  7
<212>  PRT
<213>  Homo sapiens

<400>  17

Gly Ser Gly Arg Tyr Tyr Asp
1               5


<210>  18
<211>  6
<212>  PRT
<213>  Homo sapiens

<400>  18

Ser Gly Arg Tyr Tyr Asp
1               5


<210>  19
<211>  3
<212>  PRT
<213>  Homo sapiens

<400>  19

Asp Glu Gln
1


<210>  20
<211>  15
<212>  PRT
<213>  Homo sapiens

<400>  20

Thr Gln Arg Ser Arg Gly Phe Gly Phe Ile Thr Phe Thr Asn Pro
1               5                   10                  15


<210>  21
<211>  20
<212>  PRT
<213>  Homo sapiens

<400>  21

Thr Gln Arg Ser Arg Gly Phe Gly Phe Ile Thr Phe Thr Asn Pro Glu
1               5                   10                  15


His Ala Ser Val
            20


<210>  22
<211>  3
<212>  PRT
<213>  Homo sapiens

<400>  22

Phe Thr Asn
1


<210>  23
<211>  21
<212>  PRT
<213>  Homo sapiens

<400>  23

Cys Thr Gln Arg Ser Arg Gly Phe Gly Phe Ile Thr Phe Thr Asn Pro
1               5                   10                  15

Glu His Ala Ser Val
            20

<210>  24
<211>  15
<212>  PRT
<213>  Homo sapiens

<400>  24

Gly Phe Gly Phe Ile Thr Phe Thr Asn Pro Glu His Ala Ser Val
1               5                   10                  15

<210>  25
<211>  15
<212>  PRT
<213>  Homo sapiens

<400>  25

Thr Phe Thr Asn Pro Glu His Ala Ser Val Ala Met Arg Ala Met
1               5                   10                  15

<210>  26
<211>  10
<212>  PRT
<213>  Homo sapiens

<400>  26

Thr Phe Thr Asn Pro Glu His Ala Ser Val
1               5                   10

<210>  27
<211>  126
<212>  PRT
<213>  Homo sapiens

<400>  27

Asp Glu Gln Ala Leu Glu Asp His Phe Ser Ser Phe Gly Pro Ile Ser
1               5                   10                  15

Glu Val Val Val Val Lys Asp Arg Glu Thr Gln Arg Ser Arg Gly Phe
            20                  25                  30

Gly Phe Ile Thr Phe Thr Asn Pro Glu His Ala Ser Val Ala Met Arg
        35                  40                  45

Ala Met Asn Gly Glu Ser Leu Asp Gly Arg Gln Ile Arg Val Asp His
    50                  55                  60

Ala Gly Lys Ser Ala Arg Gly Thr Arg Gly Gly Gly Phe Gly Ala His
65                  70                  75                  80

Gly Arg Gly Arg Ser Tyr Ser Arg Gly Gly Gly Asp Gln Gly Tyr Gly
                85                  90                  95

Ser Gly Arg Tyr Tyr Asp Ser Arg Pro Gly Gly Tyr Gly Tyr Gly Tyr
            100                 105                 110

Gly Arg Ser Arg Asp Tyr Asn Gly Arg Asn Gln Gly Gly Tyr
        115                 120                 125

<210>  28
<211>  77
<212>  PRT
<213>  homo sapiens

<400>  28

Thr Gln Arg Ser Arg Gly Phe Gly Phe Ile Thr Phe Thr Asn Pro Glu
1               5                   10                  15

His Ala Ser Val Ala Met Arg Ala Met Asn Gly Glu Ser Leu Asp Gly
            20                  25                  30

Arg Gln Ile Arg Val Asp His Ala Gly Lys Ser Ala Arg Gly Thr Arg
        35                  40                  45

Gly Gly Gly Phe Gly Ala His Gly Arg Gly Arg Ser Tyr Ser Arg Gly
    50                  55                  60

Gly Gly Asp Gln Gly Tyr Gly Ser Gly Arg Tyr Tyr Asp
65                  70                  75

## Description

### Field of the present disclosure

The present invention relates to the field of treatment prediction, in particular to the prediction of the response to treatment with gemcitabine.

### Background

### Cancer

Cancer is one of the most common diseases, and a major cause of death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of biopsy material from suspected tumors remains the golden standard for cancer diagnostics. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient. A commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be involved in supporting the accurate diagnosis, including staging and grading, of a primary tumor as well as in the diagnostics of metastases of unknown origin. The most commonly used antibodies in clinical practice today include antibodies against cell type "specific" proteins, e.g., PSA (prostate), MelanA (melanocytes) and Thyroglobulin (thyroid gland), and antibodies recognizing intermediate filaments (epithelial, mesenchymal, glial), cluster of differentiation (CD) antigens (hematopoetic, sub-classification of lympoid cells) and markers of malignant potential, e.g., Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites adds relevant information. However, most of these analyses still represent basic research and have yet to be evaluated and standardized for the use in clinical medicine.

### Gemcitabine treatment

Gemcitabine is a nucleoside analogue that is used in chemotherapy treatment of several cancers (e.g urothelial-, pancreatic-, lung- and breast cancer). Common side effects include fever, fatigue, nausea, vomiting, and low blood counts. Gemcitabine is metabolized within the cell, resulting in active diphosphate (dFdCDP)- and triphosphate (dFdCTP) nucleosides. dFdCDP inhibits ribonucleotide reductase, the catalyst in the production of deoxynucleoside triphosphates (dCTP) for DNA-synthesis. Inhibiting this enzyme reduces the concentration of deoxynucleosides in general, and in particular dCTP. Also, dFdCTP competes with dCTP for DNA incorporation. The reduced concentration of dCTP leads to an increased amount of dFdCTP being incorporated into the DNA strand. DNA polymerase epsilon lacks the ability to remove the faulty nucleoside, and the DNA strand cannot be repaired. This inhibits DNA synthesis leading to apoptosis. Gemcitabine may also to some extent be incorporated into RNA.

### Endpoint analysis

Endpoint analysis for trials with adjuvant treatments for cancer gives important information on how the patients respond to a certain therapy. Overall survival (OS) has long been considered the standard primary endpoint. OS takes in to account time to death, irrespective of cause, e.g. if the death is due to cancer or not. Loss to follow-up is censored and regional recurrence, distant metastases, second primary cancers and second other primary cancers are ignored.

Today, an increasing number of effective treatments available for many types of cancer have resulted in the need for surrogate endpoints to allow for a better evaluation of the effect of adjuvant treatments. Partly due to the long follow-up period required to demonstrate that adjuvant treatments improve OS, the endpoint is often complemented with other clinical endpoints that give an earlier indication on how successful the treatment is.

In the present disclosure, the inventors show that the level of expression of a particular protein (the proposed biomarker) significantly correlates with the response to gemcitabine treatment. For this observation, mainly OS is used as end-point. A surrogate endpoint of recurrence free survival (RFS) is also used. RFS includes time to any event related to the same cancer, i.e., all cancer recurrences and deaths from the same cancer are events. On the other hand, second primary same cancers and other primary cancers are ignored. Deaths from other cancers, non-cancer-related deaths, treatment-related deaths, and loss to follow-up are censored observations.

### Summary of the present disclosure

Cancer in general and advanced pancreatic cancer in particular is complex to treat. In case of pancreatic cancer, adjuvant chemotherapy will follow whenever surgical resection is possible according to many treatment recommendations in use in 2014. The adjuvant chemotherapy for pancreatic cancer commonly includes gemcitabine or 5-FU. Such adjuvant treatments have been shown to improve survival, although modestly, also for patients with periampullary tumors. Despite treatment with surgery and chemotherapy, the 5-year overall survival for patients with pancreatic adenocarcinoma is less than 5%. The death rates are thus almost identical to the incidence rates. The disease is number four among leading causes of cancer death in the United States.

There is thus a need for better molecular tools and methods to assist in an improved predictive stratification of cancer subjects, such as subjects having a pancreatic or periampullary cancer, for which gemcitabine treatment is an option.

The following is a non-limiting and itemized listing of embodiments of the present disclosure, presented for the purpose of providing various features and combinations provided by the present invention in certain of its aspects.
1. Method of determining whether a mammalian subject having a cancer belongs to a first or a second group, wherein subjects of the first group are more responsive to gemcitabine than subjects of the second group, comprising the steps of:
   a) evaluating the amount of RBM3 protein or RBM3 mRNA present in at least part of a sample derived from the cancer of said subject, and determining a sample value corresponding to said amount;
   b) comparing the sample value obtained in step a) with a reference value; and,
   if said sample value is higher than said reference value,
   c1) concluding that said subject belongs to said first group; and
   if said sample value is lower than or equal to said reference value,
   c2) concluding that said subject belongs to said second group.
2. Method for determining whether a mammalian subject having a cancer is not in need of gemcitabine treatment, comprising the steps of:
   a) evaluating the amount of RBM3 protein or RBM3 mRNA present in at least part of a sample derived from the cancer of said subject, and determining a sample value corresponding to said amount;
   b) comparing the sample value obtained in step a) with a reference value; and,
   if said sample value is lower than or equal to said reference value,
   c) concluding that the subject is not in need of gemcitabine treatment.
3. Non-treatment strategy method for a mammalian subject having a cancer, comprising the steps of:
   a) evaluating the amount of RBM3 protein or RBM3 mRNA present in at least part of a sample derived from the cancer of said subject, and determining a sample value corresponding to said amount;
   b) comparing the sample value obtained in step a) with a reference value; and,
   if said sample value is lower than or equal to said reference value,
   c) refraining from treating the subject with gemcitabine.
4. Method of treatment of a mammalian subject having a cancer, comprising the steps of:
   a) evaluating the amount of RBM3 protein or RBM3 mRNA present in at least part of a sample derived from the cancer of said subject, and determining a sample value corresponding to said amount;
   b) comparing the sample value obtained in step a) with a reference value; and,
   if said sample value is higher than said reference value,
   c) administering gemcitabine to said subject.
5. A method of determining whether to treat a mammalian subject having a cancer with gemcitabine, comprising the steps of:
   a) evaluating the amount of RBM3 protein or RBM3 mRNA present in at least part of a sample derived from the cancer of said subject, and determining a sample value corresponding to said amount;
   b) comparing the sample value obtained in step a) with a reference value; and,
   if said sample value is higher than said reference value,
   c1) administering gemcitabine to said subject, and
   if said sample value is lower than or equal to said reference value,
   c1) refraining from administering gemcitabine to said subject.
6. Method according to any one of the preceding items, wherein said cancer is selected from the group consisting of non-small cell lung cancer, pancreatic cancer, urothelial cancer, breast cancer, esophageal cancer, lymphomas, pancreatic and periampullary cancers.
7. Method according to any one of the preceding items, wherein said cancer is selected from the group consisting of duodenal cancer, ampullary cancer, distal bile duct cancer and pancreatic cancer.
8. Method according to any one of the preceding items, wherein said cancer is selected from the group consisting of distal bile duct cancer, pancreatic cancer and ampullary cancer of the pancreatobiliary type.
9. Method according to any one of the preceding items, wherein said sample is a tumor tissue sample.
10. Method according to item 9, wherein the evaluation of step a) is limited to the nuclei of tumor cells of said sample.
11. Method according to any one of the preceding items, wherein said reference value is a nuclear fraction, a nuclear intensity or a function of a nuclear fraction and a nuclear intensity.
12. Method according to item 11, wherein said reference value is a nuclear fraction of 50-90 %.
13. Method according to item 11, wherein said reference value is a moderate nuclear intensity.
14. Method according to any one of the preceding items, wherein step a) comprises:
   aI) applying to said sample a quantifiable affinity ligand capable of selective interaction with the RBM3 protein to be evaluated, said application being performed under conditions that enable binding of the affinity ligand to RBM3 protein present in the sample; and
   aII) quantifying the affinity ligand bound to said sample to evaluate said amount.
15. Method according to any one of items 1-13, wherein step a) comprises:
   a1) applying to said sample a quantifiable affinity ligand capable of selective interaction with the RBM3 protein to be quantified, said application being performed under conditions that enable binding of the affinity ligand to RBM3 protein present in the sample;
   a2) removing non-bound affinity ligand; and
   a3) quantifying affinity ligand remaining in association with the sample to evaluate said amount.
16. Method according to item 14 or 15, wherein the quantifiable affinity ligand is selected from the group consisting of antibodies, fragments thereof and derivatives thereof.
17. Method according to item 16, wherein the antibody fragments and derivatives are selected from the group consisting of Fab fragments, Fv fragments and single chain Fv (scFv) fragments.
18. Method according to item 17, wherein said quantifiable affinity ligand is obtainable by a process comprising a step of immunizing an animal with a peptide whose amino acid sequence consists of a sequence selected from the group consisting of SEQ ID NO:1, SEQ ID NO:4, SEQ ID NO:5, SEQ ID NO:27 and SEQ ID NO:28.
19. Method according to item 17 or 18, wherein said quantifiable affinity ligand is obtainable by a process comprising a step of immunizing an animal with an RBM3 fragment which consists of 21 amino acid residues or less, such as 15 amino acid residues or less, and comprises a sequence selected from the group consisting of SEQ ID NO:6-19 and 22-26.
20. Method according to any one of items 17-19, wherein said quantifiable affinity ligand is obtainable by a process comprising a step of immunizing an animal with an RBM3 fragment which consists of 20 amino acid residues or less, such as 15 amino acid residues or less, such as 10 amino acid residues or less and comprises a sequence selected from SEQ ID NO:8, 16, 17, 22, 24, 25 and 26.
21. Method according to item 14 or 15, wherein said quantifiable affinity ligand is an oligonucleotide molecule.
22. Method according to item 14 or 15, wherein the quantifiable affinity ligand is a protein ligand derived from a scaffold selected from the group consisting of staphylococcal protein A and domains thereof, lipocalins, ankyrin repeat domains, cellulose binding domains, y crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
23. Method according to any one of items 14-22, wherein said quantifiable affinity ligand is capable of selective interaction with a peptide whose amino acid sequence consists of SEQ ID NO: 1.
24. Method according to any one of items 14-23, wherein said quantifiable affinity ligand is capable of selective interaction with a peptide consisting of an amino acid sequence selected from SEQ ID NO: 4 and 5.
25. Method according to any one of items 14-24, wherein said quantifiable affinity ligand is capable of selective interaction with an RBM3 fragment which consists of 21 amino acid residues or less, such as 15 amino acid residues or less, and comprises an amino acid sequence selected from SEQ ID NO: 6-19 and 22-26.
26. Method according to any one of items 14-25, wherein said quantifiable affinity ligand is capable of selective interaction with an RBM3 fragment which consists of 20 amino acid residues or less, such as 15 amino acid residues or less, and comprises a sequence selected from SEQ ID NO: 8, 16, 17, 22, 24, 25 and 26.
27. Method according to any one of items 14-26, wherein said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
28. Method according to any one of items 14-27, wherein said quantifiable affinity ligand is detected using a secondary affinity ligand capable of recognizing said quantifiable affinity ligand.
29. Method according to item 28, wherein said secondary affinity ligand capable of recognizing said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
30. Use *ex vivo* of an RBM3 protein or RBM3 mRNA molecule as a marker of response to gemcitabine treatment of a mammalian subject having a cancer, wherein the protein or mRNA molecule is provided in a sample derived from the cancer of the subject.
31. Use according to item 30, wherein the sample is a tumor tissue sample.
32. Use *ex vivo* of an affinity ligand capable of selective interaction with an RBM3 protein for predicting the response to a gemcitabine treatment of a mammalian subject having a cancer.
33. Use *ex vivo* of an affinity ligand capable of selective interaction with an RBM3 protein as a treatment predictive agent for gemcitabine treatment of a mammalian subject having a cancer.
34. Use *ex vivo* of an affinity ligand capable of selective interaction with an RBM3 protein for indicating whether a mammalian subject having a cancer should be given a gemcitabine treatment.
35. Use according to any one of items 32-34, wherein the affinity ligand is selected from the group consisting of antibodies and Fab fragments, Fv fragments and single chain Fv (scFv) fragments thereof.
36. Use according to any one of items 32-35, wherein said affinity ligand is capable of selective interaction with a peptide whose amino acid sequence consists of SEQ ID NO: 1.
37. Use according to any one of items 32-36, wherein said affinity ligand is capable of selective interaction with a peptide consisting of an amino acid sequence selected from SEQ ID NO: 4 and 5.
38. Use according to any one of items 32-37, wherein said affinity ligand is capable of selective interaction with an RBM3 fragment which consists of 21 amino acid residues or less, such as 15 amino acid residues or less, and comprises an amino acid sequence selected from SEQ ID NO: 6-19 and 22-26.
39. Use according to any one of items 32-38, wherein said affinity ligand is capable of selective interaction with an RBM3 fragment which consists of 20 amino acid residues or less, such as 15 amino acid residues or less, and comprises a sequence selected from SEQ ID NO: 8, 16, 17, 22, 24, 25 and 26.
40. Use according to any one of items 30-39, wherein the cancer is selected from the group consisting of non-small cell lung cancer, pancreatic cancer, urothelial cancer, breast cancer, esophageal cancer, lymphomas, pancreatic and periampullary cancers.
41. Use according to any one of items 30-40, wherein the cancer is selected from the group consisting of duodenal cancer, ampullary cancer, distal bile duct cancer and pancreatic cancer.
42. Use according to any one of items 30-41, wherein the cancer is selected from the group consisting of distal bile duct cancer, pancreatic cancer and ampullary cancer of the pancreatobiliary type.
43. Gemcitabine for use in a method of treatment of a mammalian subject having a cancer, wherein said method comprises the step of determining that an amount of RBM3 protein or RBM3 mRNA in a sample derived from the cancer of the subject is higher than a relevant reference value.
44. Gemcitabine for use according to item 43, wherein the cancer is selected from the group consisting of non-small cell lung cancer, pancreatic cancer, urothelial cancer, breast cancer, esophageal cancer, lymphomas, pancreatic and periampullary cancers.
45. Gemcitabine for use according to item 43 or 44, wherein the cancer is selected from the group consisting of duodenal cancer, ampullary cancer, distal bile duct cancer and pancreatic cancer.
46. Gemcitabine for use according to item 45, wherein the cancer is selected from the group consisting of distal bile duct cancer, pancreatic cancer and ampullary cancer of the pancreatobiliary type.

### Brief description of the figures

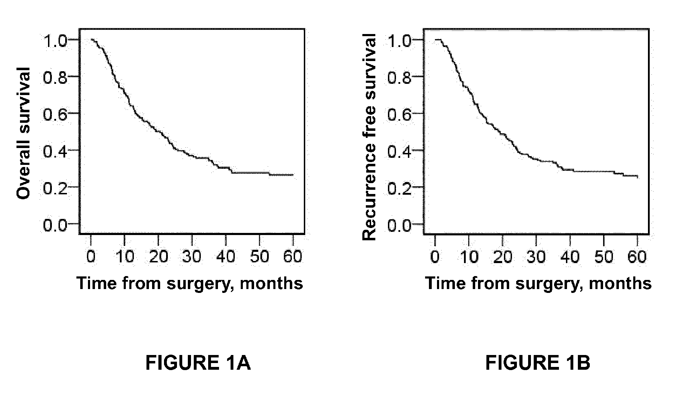

Figure 1 shows the results of survival analyses of all patients, i.e. 175 subjects. Figure 1A shows overall survival (OS), and Figure 1B shows recurrence free survival (RFS). Estimated five-year survival is approximately 28% for the full cohort, irrespective of end-point (OS or RFS).

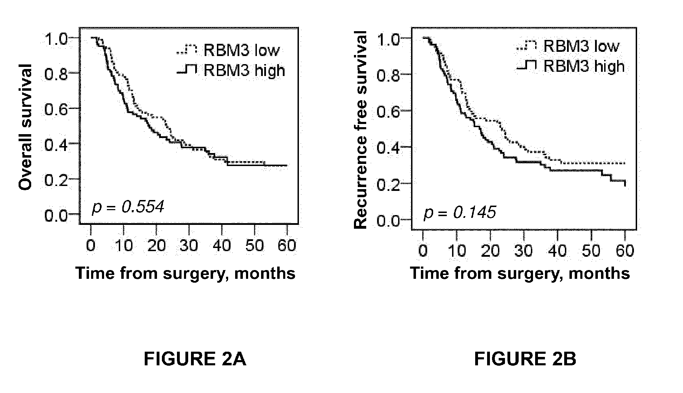

Figure 2 shows survival analysis of all patients included for further statistical analysis (n = 166). Figure 2A shows OS and Figure 2B shows RFS. Patients were split into two groups based on RBM3 protein expression in the tumors. A cut-off of NS = 1.77 was used and a solid line represents a high level of RBM3 protein expression (NS > 1.77, n = 83), a dotted line represents a low level of RBM3 protein expression (NS ≤ 1.77, n = 83).

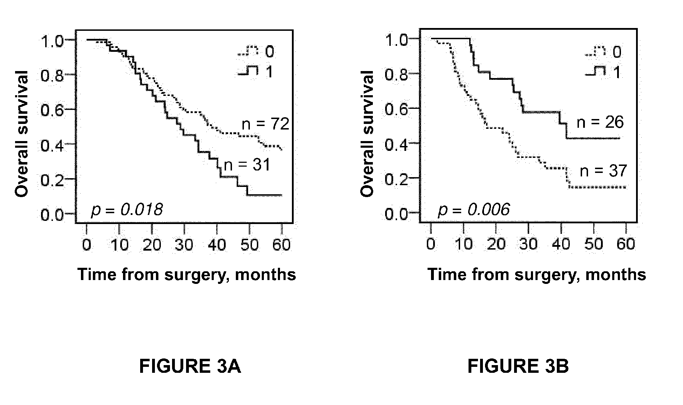

Figure 3 shows the impact of gemcitabine treatment on OS for patients with RBM3 low (Figure 3A) and RBM3 high (Figure 3B) tumors, respectively. A cut-off of NS = 2.0 was used for stratifying patients according to RBM3 protein expression. Patients were further divided into two groups: Those receiving gemcitabine as adjuvant treatment, and those receiving no- or other adjuvant treatment. The solid line represents patients treated with gemcitabine, and the dotted line represents patients not treated with gemcitabine.

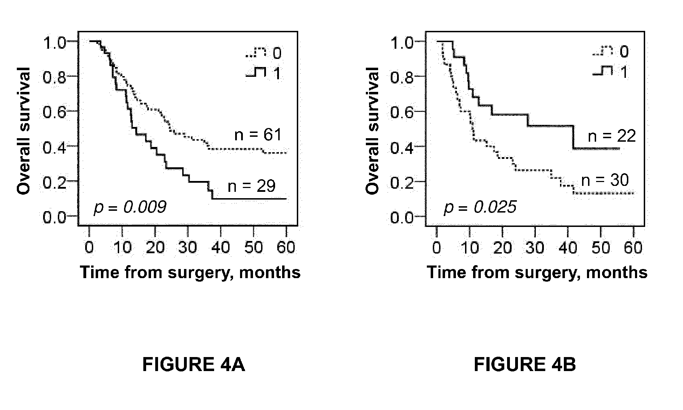

Figure 4 shows impact of gemcitabine treatment on OS for patients with RBM3 low (Figure 4A) and RBM3 high (Figure 4B) tumors, respectively. A cut-off of NS = 2.0 was used for stratifying patients according to RBM3 expression. Patients receiving only other forms of adjuvant therapy were excluded from the analysis. The solid line represents patients treated with gemcitabine, and the dotted line represents patients not receiving adjuvant treatment.

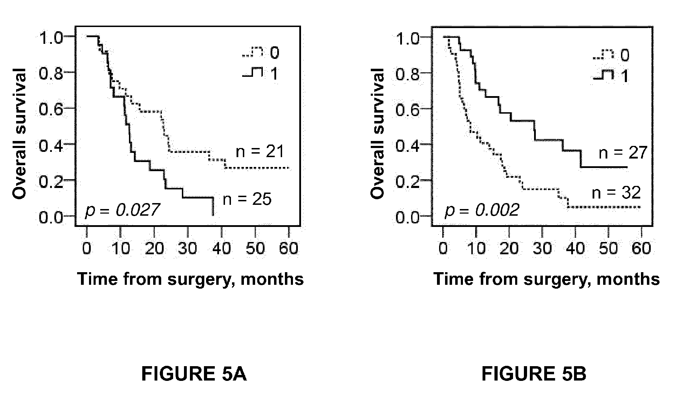

Figure 5 shows OS for patients with the pancreatobiliary subtype.
Figure 5A shows impact of gemcitabine treatment on OS for patients with RBM3 low tumors (NS ≤ 1.77), and Figure 5B shows impact of gemcitabine treatment on OS for patients with RBM3 high tumors (NS > 1.77). The solid line represents patients treated with gemcitabine, and the dotted line represents patients not treated with gemcitabine.

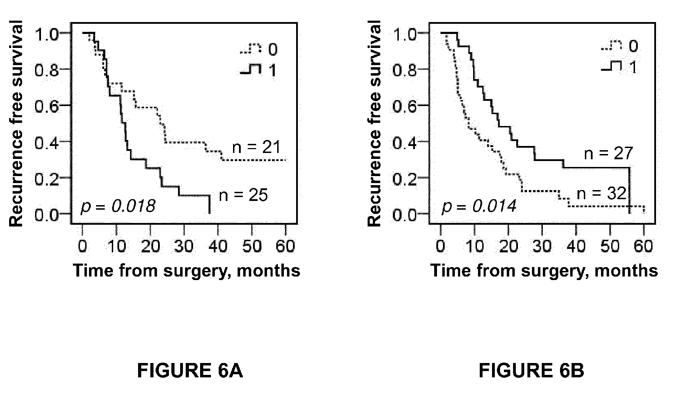

Figure 6 shows RFS for patients with the pancreatobiliary subtype. Figure 6A shows impact of gemcitabine treatment on RFS for patients with RBM3 low tumors (NS ≤ 1.77), and Figure 6B shows impact of gemcitabine treatment on RFS for patients with RBM3 high tumors (NS > 1.77). The solid line represents patients treated with gemcitabine, and the dotted line represents patients not treated with gemcitabine.

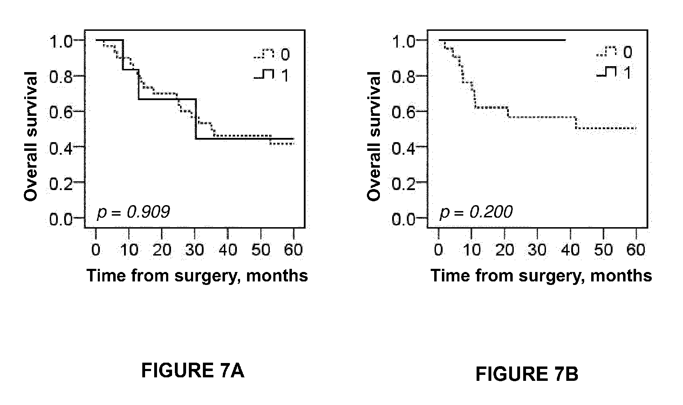

Figure 7 shows OS for patients with the intestinal subtype. Figure 7A shows impact of gemcitabine treatment on OS for patients with RBM3 low tumors (NS ≤ 1.77, n = 37). The solid line represents patients treated with gemcitabine (n = 6), and the dotted line represents patients not treated with gemcitabine (n = 31). Figure 7B shows impact of gemcitabine treatment on OS for patients with RBM3 high tumors (NS > 1.77, n = 24). The solid line represents patients treated with gemcitabine (n = 3), and the dotted line represents patients not treated with gemcitabine (n = 21).

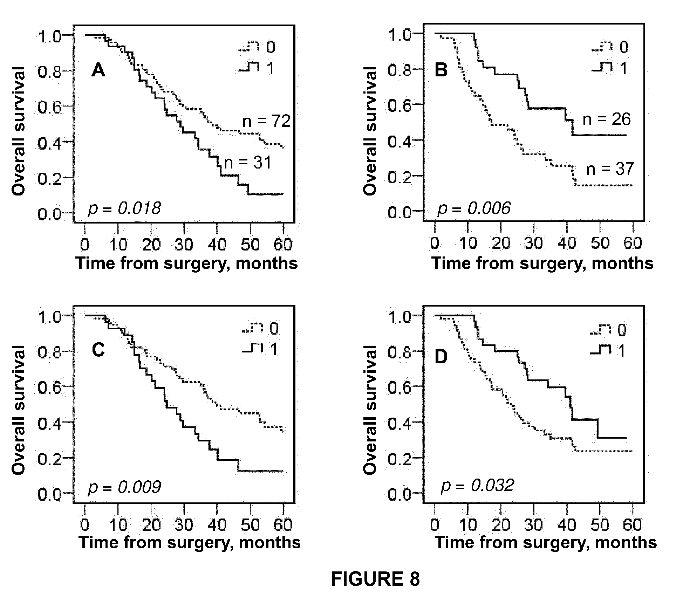

Figure 8 shows impact of gemcitabine treatment on OS for patients with RBM3 low (A and C) and RBM3 high (B and D) tumors respectively. Two different cut-offs were used for stratifying patients according to RBM3 expression. In A and B, a cut-off of NS = 1.77 were used and in C and D, a cut-off of NS = 2.0 were used.

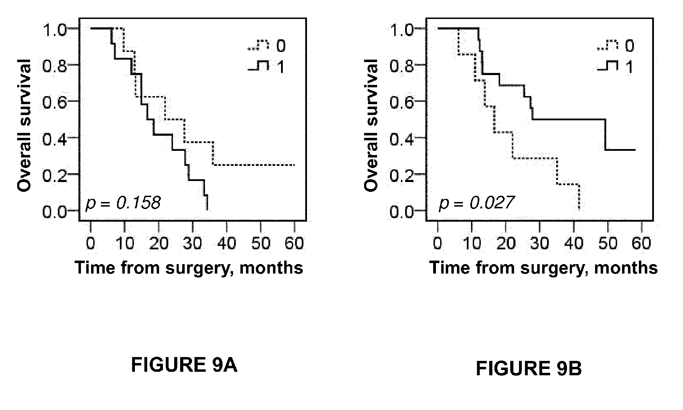

Figure 9 shows OS for patients with pancreatic adenocarcinoma. Figure 9A shows impact of gemcitabine treatment on OS for patients with RBM3 low tumors (NS ≤ 1.77, n = 20). The solid line represents patients treated with gemcitabine (n = 12), and the dotted line represents patients not treated with gemcitabine (n = 8). Figure 9B shows impact of gemcitabine treatment on OS for patients with RBM3 high tumors (NS > 1.77, n = 23). The solid line represents patients treated with gemcitabine (n = 16), and the dotted line represents patients not treated with gemcitabine (n = 7).

### Detailed description

As a first aspect of the present disclosure, there is provided a method of determining whether a mammalian subject having a cancer belongs to a first or a second group, wherein subjects of the first group are more responsive to gemcitabine than subjects of the second group, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is higher than said reference value,
c1) concluding that said subject belongs to said first group; and
if said sample value is lower than or equal to said reference value,
c2) concluding that said subject belongs to said second group.

It is thus determined whether a cancer subject belongs to a first or a second group, wherein subjects of the first group generally are more responsive to gemcitabine than subjects of the second group. The division of subjects having a given cancer into the two groups is determined by comparing samples values from the subjects with a reference value. The reference value is thus the determinant for the size of the respective groups; the higher the reference value, the fewer the subjects in the first group and the lower the likelihood that a tested subject belongs to the first group.

For the avoidance of doubt, the first and the second group consist of subjects having the same type of cancer as the tested subject. Pancreas cancer subjects are thus not compared to or grouped with subjects having lung cancer. However, a subject having a cancer of the pancreatobiliary type may be compared to and grouped with other subjects having cancers of the pancreatobiliary type. Further, the first and the second group may consist of subjects having the same or similar stage and/or subtype of cancer as the tested subject. Also, the groups may consist of subjects having the same or similar age, race, sex, menopausal status, genetic characteristics and/or medical status or history as the tested subject.

The present disclosure is based on the inventors' insight that the expression of RBM3 in a sample obtained from a subject having a cancer may serve as an indicator of the subject's response to gemcitabine. The inventors have identified a correlation between values of RBM3 on the one hand and response to gemcitabine treatment on the other. Typically, high RBM3 values are shown herein to correlate with a relatively high responsiveness to gemcitabine.

Gemcitabine has proven effective, in particular in pancreatic cancer, but also in non-small cell lung cancer, urothelial cancer and breast cancer. Further, it is being investigated for use in esophageal cancer, and is used experimentally in lymphomas and various other tumor types.

Gemcitabine treatment is however associated with several side effects, such as:
low white blood cell count with increased risk of infection;
low platelet count with increased risk of bleeding;
low red blood cell count (anemia) with symptoms like tiredness, weakness, or shortness of breath;
nausea;
vomiting;
loss of appetite;
fatigue;
fever;
swelling arms and legs or other parts of the body;
skin rash

Therefore, the inventors have concluded that there is a value in identifying the subjects who are unlikely to respond positively to (or at all benefit from) the treatment. Such subjects should be treated with alternative regimens.

In conclusion, the methods of the present disclosure may assist the physician responsible for the treatment of a cancer subject when deciding whether or not to apply gemcitabine.

It follows that an analysis of RBM3 may be used for deciding to refrain from gemcitabine treatment. As a first configuration of a second aspect of the present disclosure, there is thus provided a method for determining whether a mammalian subject having a cancer is not in need of gemcitabine treatment, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is lower than or equal to said reference value,
c) concluding that the subject is not in need of gemcitabine treatment.
As a second configuration of the second aspect, there is provided a non-treatment strategy method for a mammalian subject having a cancer, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is lower than or equal to said reference value,
c) refraining from treating the subject with gemcitabine.

In any case, RBM3 high cancer subjects are likely to benefit from gemcitabine according to teachings of the present disclosure. As a third aspect of the present disclosure, there is thus provided a method of treatment of a mammalian subject having a cancer, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is higher than said reference value,
c) administering gemcitabine to said subject.

The method of treatment may be limited to the decision-making and treatment. As a variant of the third aspect, there is thus provided a method of treatment of a subject having a cancer, comprising:
α) comparing a sample value corresponding to a level of RBM3 protein or RBM3 mRNA in a sample derived from the cancer with a reference value; and,
if said sample value is higher than said reference value,
β) administering gemcitabine to said subject.

Numerous ways of obtaining a sample value corresponding to a level of RBM3 protein or RBM3 mRNA in a sample from a subject are described in the present disclosure, which also provides examples of clinically relevant reference values.

A configuration of the third aspect follows from the discussion above.

According to the configuration, there is provided a method of determining whether to treat a mammalian subject having a cancer with gemcitabine, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is higher than said reference value,
c1) administering gemcitabine to said subject, and
if said sample value is lower than or equal to said reference value,
c1) refraining from administering gemcitabine to said subject.

Regarding step a) of the methods of the present disclosure, an increase in the amount of RBM3 protein or RBM3 mRNA typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of RBM3 protein or RBM3 mRNA will not result in a decrease in the sample value in the context of the present disclosure.

However inconvenient, but in an equivalent fashion, the evaluated amounts may be inversely related to sample values if the qualification between step b) and c) is inverted. For example, the qualification between step b) and step c) is inverted if the phrase "if the sample value is higher than the reference value" is replaced with "if the sample value is lower than the reference value".

A physician responsible for the treatment of a cancer subject may take several parameters into account, such as the result of an immunohistochemical evaluation, patient age, hormone receptor status, general condition of the patient, medical history, such as cancer history and hereditary characteristics, e.g. whether there is a history of cancer in the subject's family when deciding on a suitable treatment regimen for the subject. To be guided in the decision, the physician may perform an RBM3 test, or order an RBM3 test performed, according to anyone of the above methods. Further, the physician may assign to someone else, such as a lab worker, to perform step a), and optionally step b), while performing step c), and optionally b), himself.

Step a) of the methods of the present disclosure may be carried out ex *vivo,* which for example means that the step of obtaining a sample from the subject is not part of the methods.

In the context of the present disclosure, "a mammalian subject having a cancer" refers to a mammalian subject having a primary or secondary tumor or a mammalian subject which has had such tumor removed, wherein the removal of the tumor refers to killing or removing the tumor by any type of surgery or therapy. In the latter case, the tumor may for example have been removed less than one year ago. For example, a subject who has had a tumor removed by surgery and is about to get adjuvant therapy is considered "having a cancer" in the context of the present disclosure.

Further, in the context of the present disclosure, the "reference value" refers to a predetermined value which is relevant for making decisions, or drawing conclusions, regarding the treatment or treatment prediction. Guided by the teachings of the present disclosure, the person skilled in the art may select a relevant reference value without undue burden.

Step a) of the methods of the above aspects involve evaluating an amount of RBM3 present in at least part of the sample, and determining a sample value corresponding to the amount. The "at least part of the sample" refers to a relevant part or relevant parts of the sample for drawing conclusions regarding a suitable treatments. The person skilled in the art understands which part or parts that are relevant under the circumstances present when performing the method. For example, the skilled person may only consider the nuclei of tumor cells of the sample. Likewise, an automated analysis, such as an automated scoring system for stained tissue samples, may be adapted to take only the relevant part(s) into account.

Further, in step a) an amount is evaluated and a sample value corresponding to the amount is determined. Consequently, an exact measurement of the amount of RBM3 protein or RBM3 mRNA is not required for obtaining the sample value. For example, the amount of RBM3 protein may be evaluated by visual inspection of a prepared and stained tissue sample and the sample value may then be categorized as for example "high" or "low" based on the evaluated amount.

The cancer of the present disclosure may for example be selected from the group consisting of non-small cell lung cancer, urothelial cancer (such as bladder cancer), breast cancer, esophageal cancer, lymphomas, pancreatic cancer and periampullary cancers.

A periampullary cancer is a cancer that forms near the ampulla of Vater, an enlargement of the ducts from the liver and pancreas where they join and enter the small intestine. Periampullary cancers comprise ampullary, distal bile duct, pancreatic, and duodenal cancers.

The Example below describes an analysis of a cohort of subjects having tumors of the following origins: duodenum, papilla-ampulla intestinal type, papilla-ampulla pancreatobiliary type, distal bile duct and pancreas. The cohort may be divided into two subgroups: subjects having intestinal-type tumors, which include duodenum and papilla-ampulla intestinal type; and subjects having pancreatobiliary tumors, which include papilla-ampulla pancreatobiliary type, distal bile duct and pancreas.

Figures 5-7 indicate that RBM3 is a predictor of response to gemcitabine in both subgroups. The association between high RBM3 expression and response to gemcitabine is however particularly accentuated in latter subgroup (see figures 5-6). Accordingly, the cancer of the present disclosure may for example be selected from the group consisting of distal bile duct cancer, pancreatic cancer and ampullary cancer of the pancreatobiliary type.

In the methods of the present disclosure, the level of RBM3 protein is preferably measured intracellularly or in cell-derived material. Thus, the sample preferably comprises tumor cells. Accordingly, the sample may be a tumor tissue sample.

RBM3 protein expression is both nuclear and cytoplasmic. The inventors have however found that the nuclear expression is particularly relevant for making the treatment predictions of the present disclosure.

The evaluation of step a) may thus be limited to the nuclei and/or cytoplasms (preferably the nuclei only) of tumor cells of said sample. Consequently, when a tissue sample is examined, only the nuclei of tumor cells may be taken into consideration. Such examination may for example be aided by immunohistochemical staining.

The quantification of RBM3 in the nuclei of cells is discussed below.

A sample value of RBM3 protein or RBM3 mRNA being higher than the reference value, or a subject from which such sample value is obtained, is sometimes referred to herein as being "RBM3 high". Further, a sample value of RBM3 protein or RBM3 mRNA being lower than, or equal to, the reference value, or a subject from which such sample value is obtained, is sometimes referred to herein as being "RBM3 low".

In the context of the present disclosure, the terms "sample value" and "reference value" are to be interpreted broadly. The quantification of RBM3 to obtain these values may be done via automatic means, via a scoring system based on visual or microscopic inspection of samples, or via combinations thereof. However, it is also possible for a skilled person, such as a person skilled in the art of histopathology, to determine the sample and reference values by inspection, e.g., of tissue slides that have been prepared and stained for RBM3 protein expression.

Determining that the sample value is higher than the reference value may thus be determining, upon visual or microscopic inspection, that a sample tissue slide is more densely stained and/or exhibit a larger fraction of stained cells than a reference tissue slide. The sample value may also be compared to a reference value given by a literal reference, such as a reference value described in wording or by a reference picture. Consequently, the sample and/or reference values may in some cases be mental values that the skilled person envisages upon inspection and comparison.

One or more of the steps of the methods of the present disclosure may be implemented in an apparatus. For example, step a) and optionally step b) may be performed in an apparatus for automatic staining and/or analysis, and such an apparatus may be based on a platform adapted for immunohistochemical staining and/or analysis. As an example, one or more tumor tissue sample(s) from the subject in question may be prepared for imunohistochemical staining and/or analysis manually and then loaded into the apparatus for automatic staining and/or analysis, which gives the sample value of step a) and optionally also performs the comparison with the reference value of step b). The operator performing the analysis, the physician ordering the analysis or the apparatus itself may then draw the conclusion of step c). Consequently, software adapted for drawing the conclusion of step c) may be implemented on the apparatus.

A reference value, found to be relevant for establishing treatment prediction or making treatment decisions regarding cancer subjects, for use as comparison with the sample value from the subject, may be provided in various ways. With the knowledge of the teachings of the present disclosure, the skilled artisan can, without undue burden, provide relevant reference values for performing the methods of the present disclosure.

The person performing the methods of the above aspects may, for example, adapt the reference value to desired information. For example, the reference value may be adapted to yield the most significant information with regard to survival, e.g., the largest separation between the RBM3 high survival curve and the RBM3 low survival curve (see the figures), which corresponds to the largest difference in survival between the first and the second group of the first aspect. Alternatively, the reference value may be selected such that a group of subjects having particularly high responsiveness or particularly low responsiveness is singled out.

In embodiments of the methods of the above aspects, the reference value may correspond to the amount of RBM3 protein or RBM3 mRNA measured in a reference sample comprising tumor cells, such as a reference sample of tumor tissue. Such as sample may be derived from another subject having the same type of cancer as the subject of the method. Further, the reference value may for example be provided by the amount of RBM3 protein or RBM3 mRNA measured in a reference sample comprising cancer cell lines expressing a predetermined, or controlled, amount of RBM3 protein or RBM3 mRNA. The person skilled in the art understands how to provide such cell lines, for example guided by the disclosure of Rhodes et al. (2006) The biomedical scientist, p 515-520.

The amount of protein or mRNA expression of the reference sample is preferably previously established.

Consequently, the reference value may be provided by the amount of RBM3 protein or RBM3 mRNA measured in a reference sample comprising cells expressing a predetermined amount of RBM3 protein or RBM3 mRNA.

As further discussed above and below, the amount of RBM3 protein or RBM3 mRNA in the reference sample does not have to directly correspond to the reference value. The reference sample may also provide an amount of RBM3 protein or RBM3 mRNA that helps a person performing the method to assess various reference values. For example, the reference sample(s) may help in creating a mental image of the reference value by providing a "positive" reference value and/or a "negative" reference value.

One alternative for the quantification of RBM3 protein expression in a sample, such as the sample of step a) or the reference sample of the above methods, is the determination of the fraction of cells in the sample that exhibit RBM3 protein expression over a certain level. The fraction may for example be: a "cellular fraction", wherein the RBM3 protein expression of the whole cells is taken into account; a "cytoplasmic fraction", wherein the RBM3 protein expression of only the cytoplasms of the cells is taken into account; or a "nuclear fraction", wherein the RBM3 protein expression of only the nuclei of the cells is taken into account. The "nuclear fraction" corresponds to the percentage of relevant cells in a sample that exhibits a positive staining in the nucleus, wherein a medium or distinct and strong immunoreactivity in the nucleus is considered positive and no or faint immunoreactivity in the nucleus is considered negative. The person skilled in the art of pathology understands which cells that are relevant under the conditions present when performing the method and may determine a nuclear fraction based on his/her general knowledge and the teachings of the present disclosure. The relevant cells may for example be tumor cells. Further, the skilled artisan understands how to perform corresponding measurements employing the "cytoplasmic fraction" or "cellular fraction".

Another alternative for the quantification of RBM3 protein expression in a sample, such as the sample of step a) or the reference sample of the above methods, is the determination of the overall staining intensity of the sample. The intensity may for example be: a "cellular intensity", wherein the RBM3 protein expression of the whole cells is taken into account; a "cytoplasmic intensity", wherein the RBM3 protein expression of only the cytoplasms of the cells is taken into account, or a "nuclear intensity", wherein the RBM3 protein expression of only the nuclei of the cells is taken into account. Nuclear intensity is subjectively evaluated in accordance with standards used in clinical histopathological diagnostics. Outcome of a nuclear intensity determination may be classified as: negative = no overall immunoreactivity in the nuclei of relevant cells of the sample, weak = faint overall immunoreactivity in the nuclei of relevant cells of the sample, moderate = medium overall immunoreactivity in the nuclei of relevant cells of the sample, or strong = distinct and strong overall immunoreactivity in the nuclei of relevant cells of the sample. The person skilled in the art understands which cells that are relevant under the conditions present when performing the method and may determine a nuclear intensity based on his general knowledge and the teachings of the present disclosure. The relevant cells may for example be tumor cells. Further, the skilled artisan understands how to perform corresponding measurements employing the "cytoplasmic intensity" or "cellular intensity".

As discussed above, the inventors have found that nuclear expression of RBM3 protein is particularly relevant for predicting the outcome of gemcitabine treatment.

Thus, in embodiments of the methods of the above aspects, the reference value may be a nuclear fraction, a nuclear intensity or a combination or function thereof. Accordingly, the sample value may be a nuclear fraction, a nuclear intensity or a combination or function thereof.

Thus, in embodiments of the methods of the above aspects, the reference value of step b) may be a nuclear fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Further, in embodiments of the methods of the above aspects the reference value of step b) may be a moderate nuclear intensity of RBM3 protein expression or lower, such as a weak nuclear intensity of RBM3 protein expression or lower, such as a negative nuclear of RBM3 protein expression.

A reference value in the higher range has been found to be particularly preferred. Thus, the reference value may preferably be a nuclear fraction of 50-90 % a moderate nuclear intensity or a function of nuclear fraction and a nuclear intensity in theses ranges.

In embodiments of the methods of the above aspects, the reference value may thus be a combination or a function of a fraction value and an intensity value (see e.g. the value used in the Example below). The reference value may thus involve two, and even more, criteria.

The reference value may thus be a nuclear score (NS), which is the product of the nuclear fraction (in the range 0-100 %) and the nuclear intensity determined as 0, 1, 2, or 3, wherein 0 is a negative nuclear intensity, 1 is a weak nuclear intensity, 2 is a moderate nuclear intensity and 3 is a strong nuclear intensity. The NS is thus the result of multiplying the nuclear fraction and the nuclear intensity and may range from 0 to 3. The reference value may for example be a NS between 1 and 2.4, such as between 1.5 and 2.2. In the Example below, a reference value/cut-off of 1.77 or 2 is used.

In general, the selection of an intensity value and/or a fraction value as the reference value may depend on the staining procedure, e.g., on the type and amount/concentration of the employed antibody and on the type and concentration of the staining reagents.

Guided by the present disclosure, a person skilled in the art, e.g., a pathologist understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of RBM3 protein for establishing the appearance of a certain fraction or intensity.

However, a reference sample may not only be used for the provision of the actual reference value, but also for the provision of an example of a sample with an amount of RBM3 that is higher than the amount corresponding to the reference value. As an example, in histochemical staining, such as in immunohistochemical staining, the skilled artisan may use a reference sample for establishing the appearance of a stained sample having a high amount of RBM3 protein, e.g., a positive reference. Subsequently, the skilled artisan may assess the appearances of samples having lower amounts of RBM3 protein, such as the appearance of a sample with an amount of RBM3 protein corresponding to the reference value. In other words, the skilled artisan may use a reference sample to create a mental image of a reference value corresponding to an amount of RBM3 protein which is lower than that of the reference sample. Alternatively, or as a complement, in such assessments, the skilled artisan may use another reference sample having a low amount of RBM3 protein, or lacking detectable RBM3 protein, for establishing the appearance of such sample, e.g., as a "negative reference".

For example, if a moderate nuclear intensity is used as the reference value, two reference samples may be employed: a first reference sample having no detectable RBM3 protein, and thus corresponding to a negative nuclear intensity, which is lower than the reference value; and a second reference sample having an amount of RBM3 protein corresponding to a strong nuclear intensity, which is higher than the reference value.

Consequently, in the evaluation, the skilled artisan may use a reference sample for establishing the appearance of a sample with a high amount of RBM3 protein. Such reference sample may be a sample comprising tissue expressing a high amount of RBM3 protein, such as a sample comprising pancreatic cancer tissue having a pre-established high expression of RBM3 protein.

Accordingly, the reference sample may provide an example of a strong nuclear intensity (NI). With the knowledge of the appearance of a sample with strong NI, the skilled artisan may then divide samples into the NI categories absent, weak, moderate and strong. This division may be further assisted by a reference sample lacking detectable RBM3 protein (negative reference), i.e., a reference sample providing an absent nuclear intensity. Also, the reference sample may provide an example of a sample with a nuclear fraction (NF) higher than 75 %. With the knowledge of the appearance of a sample with more than 75 % positive cells, the skilled artisan may then evaluate the NF of other samples having e.g., a lower percentage of positive cells. This division may be further assisted by a reference sample essentially lacking RBM3 protein (negative reference), i.e., a reference sample providing a low NF (e.g., < 5%, such as < 2%), or a NF of 0.

As mentioned above, cell lines expressing a controlled amount of RBM3 protein may be used as the reference, in particular as a positive reference.

One or more pictures may also be provided as the "reference sample". For example, such a picture may show an example of a tumor tissue slide stained with a certain antibody during certain conditions and exhibiting a certain nuclear intensity and/or fraction. The above discussion about the "reference sample" applies mutatis mutandis to pictures.

The skilled person should recognize that the present disclosure is not limited to the quantification of any particular variant of the RBM3 protein or RBM3 mRNA present in the subject in question, as long as the protein or mRNA is encoded by the relevant gene and presents the relevant pattern of expression.

The RBM3 protein of the present disclosure may for example comprise a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another example, the RBM3 protein of the present disclosure may comprise, or consists of, a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The RBM3 mRNA of the present disclosure may comprise or consists of
i) a sequence corresponding to SEQ ID NO:3 or
ii) a sequence which is at least 85 % identical to thereto.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to the sequence i).

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acids may at the most be 85 % identical to a target sequence of 100 amino acids. This corresponds *mutatis mutandis* nucleic acid sequences.

In some embodiments, step a) of the above methods may comprise:
obtaining biological material from the subject, excising or selecting a relevant part of the biological material to obtain said sample and optionally arranging the sample on a solid phase to facilitate the evaluation of step a). Step a) may thus, as an example, comprise obtaining pancreatic tumor tissue material from the subject, optionally fixating the tissue material in paraffin or formalin, histo-processing the tissue material to obtain a section which constitute said sample and optionally mounting said sample on a transparent slide, such as a glass slide, for microscopy.

In embodiments of the above methods, the RBM3 protein may be detected and/or quantified through the application to the sample of a detectable and/or quantifiable affinity ligand, which is capable of selective interaction with the RBM3 protein. The application of the affinity ligand is performed under conditions that enable binding of the affinity ligand to RBM3 protein in the sample.

To concretize, in embodiments of the methods of the aspects above, step a) may comprise:
a1) applying to said sample a quantifiable affinity ligand capable of selective interaction with the RBM3 protein to be evaluated, said application being performed under conditions that enable binding of said affinity ligand to RBM3 protein present in said sample;
a2) removing non-bound affinity ligand; and
a3) quantifying the affinity ligand remaining in association with said sample to evaluate said amount.

"Affinity ligand remaining in association with the sample" refers to affinity ligand which was not removed in step a2), e.g., the affinity ligand bound to the sample. Here, the binding may for example be the interaction between antibody and antigen.

However, in some embodiments, the removal of non-bound affinity ligand according to a2), e.g. the washing, is not always necessary. Thus, in some embodiments of the methods of the aspects above, step a) may comprise:
aI) applying to said sample a quantifiable affinity ligand capable of selective interaction with the RBM3 protein to be evaluated, said application being performed under conditions that enable binding of said affinity ligand to RBM3 protein present in said sample;
aII) quantifying the affinity bound to said sample to evaluate said amount.

In the context of the present disclosure, "selective" interaction of e.g., an affinity ligand with its target or antigen means that the interaction is such that a distinction between selective and non-selective becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high selectivity does not necessarily require high affinity. Molecules with low affinity (in the molar range) for its counterpart have been shown to be as selective as molecules with much higher affinity. In the case of the present disclosure, a selective interaction refers to the extent to which a particular method can be used to determine the presence and/or amount of a specific protein, the target protein, under given conditions in the presence of other proteins in a biological sample, such as a tissue sample or a fluid sample of a naturally occurring or processed biological fluid. In other words, specificity or selectivity is the capacity to distinguish between related proteins. For example, the specificity or selectivity of an antibody may be determined as in WO 2010/092190 (see Examples, Section 2), wherein analysis is performed using a protein array set-up, a suspension bead array and a multiplexed competition assay, respectively. Specificity and selectivity determinations are also described in Nilsson P et al. (2005) Proteomics 5:4327-4337.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below.

Thus, in embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. The polyclonal antibodies may be antigen purified. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. RBM3 protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

In some embodiments, the affinity ligand of the present disclosure is capable of selective interaction with a peptide consisting of the amino acid sequence SEQ ID NO:1. The RBM3 protein fragment SEQ ID NO:1 was designed to lack transmembrane regions to ensure efficient expression in *E. coli,* and to lack any signal peptide, since those are cleaved off in the mature protein. SEQ ID NO:1 was thus designed for immunizations. In addition, the protein fragment was designed to consist of a unique sequence with low sequence identity to other human proteins, to minimize cross reactivity of generated affinity reagents, and to be of a suitable size to allow the formation of conformational epitopes and still allow efficient cloning and expression in bacterial systems. Accordingly, in the cases wherein the affinity ligand is an antibody or fragment or derivative thereof, the affinity ligand may be obtainable by a process comprising a step of immunizing an animal with a peptide whose amino acid sequence consists of the sequence SEQ ID NO:1. For example, an immunization process may comprise primary immunization with the protein/antigen in Freund's complete adjuvant. Also, the immunization process may further comprise boosting at least two times, in intervals of 2-6 weeks, with the protein/antigen in Freund's incomplete adjuvant. Processes for the production of antibodies or fragments or derivatives thereof against a given target are known in the art.

In the context of the present disclosure, an "antigen purified antibody" is one or a population of polyclonal antibodies which has been affinity purified on its own antigen, thereby separating such antigen purified antibodies from other antiserum proteins and non-specific antibodies. This affinity purification results in antibodies that bind selectively to its antigen. In the case of the present disclosure, the polyclonal antisera are purified by a two-step immunoaffinity based protocol to obtain antigen purified antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

Polyclonal and monoclonal antibodies, as well as their fragments and derivatives, represent the traditional choice of affinity ligands in applications requiring selective biomolecular recognition, such as in the detection and/or quantification of RBM3 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific/selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against RBM3 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); y crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101(25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

As mentioned above, the RBM3 protein fragment SEQ ID NO:1 was designed to consist of a unique sequence with low sequence identity to other human proteins and to minimize cross reactivity of generated affinity reagents. Consequently, in embodiments of the present disclosure, the affinity ligand may be capable of selective interaction with a polypeptide consisting of the amino acid sequence SEQ ID NO:1.

The epitope regions SEQ ID NO:4 and 5 has been identified within SEQ ID NO:1. Thus, in some embodiments, the affinity ligand of the present disclosure is capable of selective interaction with a peptide consisting of an amino acid sequence selected from SEQ ID NO:4 and 5.

Further, another four epitope regions (SEQ ID NO:6-9) have been identified. Thus, in some embodiments, the affinity ligand of the present disclosure is capable of selective interaction with an RBM3 fragment which consists of 20 amino acid residues or less, such as 15 amino acid residues or less, and comprises a sequence selected from SEQ ID NO:6-9.

Also, another ten epitope regions (SEQ ID NO:10-19) have been identified. Thus, in some embodiments, the affinity ligand of the present disclosure is capable of selective interaction with an RBM3 fragment which consists of 20 amino acid residues or less, such as 15 amino acid residues or less, and comprises a sequence selected from SEQ ID NO:10-19.

Antibodies having selectivity for a single epitope region (such as monoclonal antibodies) may provide for increased reproducibility in detection analyses as compared to antibodies generated against a longer peptide sequence (such as a PrEST or a full-length protein). The antibodies selective for a single epitope region may also provide for distinct and strong staining in immunohistochemical analyses. These benefits, independently or jointly, may be valuable when and making treatment predictions or decisions regarding treatments according to the present disclosure.

The monoclonal antibodies named "6F11" and "1 B5" are considered to be particularly beneficial. 6F11 and 1 B5 have both been shown to be more selective than a polyclonal anti-RBM3 antibody. Further, 1 B5 has been shown to be more selective than 6F11.1 B5 is also employed in the example section below, wherein it is referred to as AAb030038.

SEQ ID NO:17, to which 1 B5 binds, is within SEQ ID NO:5. In preferred embodiments of the present disclosure, the affinity ligand is thus capable of selective interaction with an RBM3 fragment which consists of SEQ ID NO:5, and in particularly preferred embodiments of the present disclosure, the affinity ligand is capable of selective interaction with an RBM3 fragment which consists of 20 amino acid residues or less, such as 15 amino acid residues or less, and comprises the sequence SEQ ID NO:17.

6F11 has been shown to bind to SEQ ID NO:8 and SEQ ID NO:16. In other preferred embodiments of the present disclosure, the affinity ligand is thus capable of selective interaction with an RBM3 fragment which consists of 20 amino acid residues or less, such as 15 amino acid residues or less, and comprises a sequence selected from SEQ ID NO:8 and 16. Note that SEQ ID NO:8 and 16 are overlapping and that such a fragment may comprise the sequences of both SEQ ID NO:8 and 16.

In WO2013/041610, antibodies capable of selective interaction with SEQ ID NO:24 and 25 (both within SEQ ID NO:4) are shown to be particularly selective. In preferred embodiments of the present disclosure, the affinity ligand is thus capable of selective interaction with an RBM3 fragment which consists of a sequence selected from SEQ ID NO:24 and 25.

Further, the epitope regions SEQ ID NO:22 and 26 have been identified. In other preferred embodiments of the present disclosure, the affinity ligand is thus capable of selective interaction with an RBM3 fragment which comprises a sequence selected from SEQ ID NO:22 and 26 and consists of 21 amino acids or less, such as 20 amino acids or less, such as 17 amino acids or less, such as 15 amino acid residues or less, such as 10 amino acid residues or less, such as 6 amino acids or less.

In WO2013/041610 various antibodies selectively interacting with RBM3 fragments within SEQ ID NO:27 or SEQ ID NO:28 are shown to provide for better results than another antibody interacting with a RBM3 stretch outside SEQ ID NO:27 or SEQ ID NO:28.

The detection and/or quantification of the affinity ligand capable of selective interaction with the RBM3 protein may be accomplished in any way known to the skilled person for detection and/or quantification of binding reagents in assays based on biological interactions. Accordingly, any affinity ligand described above may be used to quantitatively and/or qualitatively detect the presence of the RBM3 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with RBM3 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g ³H, ¹⁴C, ³²P, ³⁵S or ¹²⁵I) and particles (e.g., gold). In the context of the present disclosure, "particles" refer to particles, such as metal particles, suitable for labeling of molecules. Such particles are well known to the skilled person. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its RBM3 protein target may involve visualizing techniques, such as light microscopy or immunofluoresence microscopy. Other methods may involve the detection via flow cytometry or luminometry.

As discussed above, biological material from the subject may be used for obtaining the sample for detection and/or quantification of RBM3 protein. The sample may thus be an earlier obtained sample. If using an earlier obtained sample in a method, no steps of the method are practiced on the human or animal body.

The affinity ligand may be applied to the sample for detection and/or quantification of the RBM3 protein. This procedure enables not only detection of RBM3 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified in order for proper detection and/or quantification.

In embodiments of the methods of the above aspects, the sample may be immobilized onto a solid phase support or carrier, such as nitrocellulose or any other solid support matrix capable of immobilizing RBM3 protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand selective for RBM3 protein may be applied. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of a1) or al) may be detected using a secondary affinity ligand capable of recognizing the quantifiable affinity ligand. The quantification of a3) or aII) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the RBM3 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the RBM3 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize RBM3 protein by detection of radioactivity *in vivo* or *ex vivo.* Radionuclear scanning with e.g., gamma camera, magnetic resonance spectroscopy or emission tomography function for detection *in vivo* and *ex vivo,* while gamma/beta counters, scintillation counters and radiographies are also used *ex vivo.*

Methods for detecting and quantifying biomarkers on the mRNA level are well known within the art.

According to one such method, total cellular RNA is purified from cells by homogenization in the presence of nucleic acid extraction buffer, followed by centrifugation. Nucleic acids are then precipitated, in order to remove DNA by treatment with DNase and precipitation. The RNA molecules are then separated by gel electrophoresis on agarose gels according to standard techniques, and transferred to nitrocellulose filters by, e.g., the so-called "Northern" blotting technique. The RNA is then immobilized on the filters by heating. Detection and quantification of specific RNA is accomplished using appropriately labeled DNA or RNA probes complementary to the RNA in question. See, for example, Molecular Cloning: A Laboratory Manual (Sambrook J.et al., (1989) 2nd edition, Cold Spring Harbor Laboratory Press). Methods for the preparation of labeled DNA and RNA probes, and the conditions for hybridization thereof to target nucleotide sequences, are described in Molecular Cloning: A Laboratory Manual (Sambrook J.et al., (1989) 2nd edition, Cold Spring Harbor Laboratory Press). For example, the nucleic acid probe may be labeled with, e.g., a radionuclide such as ³H, ³²P, ³³P, ¹⁴C, or ³⁵S; a heavy metal; or a ligand capable of functioning as a specific binding pair member for a labeled ligand (e.g., biotin, avidin, or an antibody), a fluorescent molecule, a chemi luminescent molecule, an enzyme, or the like.

Probes may be labeled to high specific activity by either the nick translation method (Rigby et al., (1977) J. Mol Biol, 113: 237-251), or by the random priming method (Fienberg, (1983) Anal. Biochem., 132: 6-13). The latter can be a method for synthesizing ³²P-labeled probes of high specific activity from RNA templates. For example, by replacing preexisting nucleotides with highly radioactive nucleotides according to the nick translation method, it is possible to prepare ³²P- labeled nucleic acid probes with a specific activity well in excess of 10 cpm/microgram. Autoradiographic detection of hybridization then can be performed by exposing hybridized filters to photographic film. Densitometric scanning of the photographic films exposed by the hybridized filters provides an accurate measurement of biomarker levels. Using another approach, biomarker levels can be quantified by computerized imaging systems, such as the Molecular Dynamics 400-B 2D Phosphorimager (Amersham Biosciences, Piscataway, NJ., USA).

Where radionuclide labeling of DNA or RNA probes is not practical, the random- primer method can be used to incorporate an analogue, for example, the dTTP analogue 5 -(N-(N-biotinyl-epsilon-aminocaproyl)-3-aminoallyl)deoxyuridine triphosphate, into the probe molecule. The biotinylated probe oligonucleotide can be detected by reaction with biotin-binding proteins, such as avidin, streptavidin, and antibodies (e.g., anti-biotin antibodies) coupled to fluorescent dyes or enzymes that produce color reactions.

In addition to Northern and other RNA blotting hybridization techniques, determining the levels of RNA transcript may be accomplished using the technique of in situ hybridization. This technique requires fewer cells than the Northern blotting technique, and involves depositing whole cells onto a microscope cover slip and probing the nucleic acid content of the cell with a solution containing radioactive or otherwise labeled nucleic acid (e.g., cDNA or RNA) probes. This technique is particularly well-suited for analyzing tissue biopsy samples from subjects.

The relative number of RNA transcripts in cells also can be determined by reverse transcription of RNA transcripts, followed by amplification of the reverse-transcribed transcripts by polymerase chain reaction (RT-PCR). The levels of RNA transcripts can be quantified in comparison with an internal standard, for example, the level of mRNA from a standard gene present in the same sample. The person skilled in the art is capable of selecting suitable genes for use as an internal standard. The methods for quantitative RT-PCR and variations thereof are within the skill in the art.

Any suitable primers can be used for the quantitative RT-PCR. Preferably, the primers are specific to RBM3. It is within the skill in the art to generate primers specific to RBM3 (e.g. starting from SEQ ID NO:3). Primers can be of any suitable length, but are preferably between 19 and 23 (e.g., 19, 20, 21, 22, or 23) nucleotides. Ideally, amplicon length should be 50 to 150 (up to 250 may be necessary but then optimization of the thermal cycling protocol and reaction components may be necessary) bases for optimal PCR efficiency. Designing primers that generate a very long amplicon may lead to poor amplification efficiency. Information about primer design and optimal amplicon size may fo example be found at www.ambion.com.

In some instances, it may be desirable to use microchip technology to detect biomarker expression. The microchip can be fabricated by techniques known in the art. For example, probe oligonucleotides of an appropriate length, e.g., 40 nucleotides, are 5 '-amine modified at position C6 and printed using commercially available microarray systems, e.g., the GENEMACHINE OmniGrid 100 Microarrayer and Amersham CODELINK activated slides. Labeled cDNA oligomer corresponding to the target RNAs is prepared by reverse transcribing the target RNA with labeled primer. Following first strand synthesis, the RNA/DNA hybrids are denatured to degrade the RNA templates. The labeled target cDNAs thus prepared are then hybridized to the microarray chip under hybridizing conditions, e.g., 6 times SSPE/30% formamide at 25 °C for 18 hours, followed by washing in 0.75 times TNT at 37 °C for 40 minutes. At positions on the array, where the immobilized probe DNA recognizes a complementary target cDNA in the sample, hybridization occurs. The labeled target cDNA marks the exact position on the array where binding occurs, thereby allowing automatic detection and quantification. The output consists of a list of hybridization events, which indicate the relative abundance of specific cDNA sequences, and therefore the relative abundance of the corresponding complementary biomarker, in the subject sample. According to one embodiment, the labeled cDNA oligomer is a biotin-labeled cDNA prepared from a biotin-labeled primer. The microarray is then processed by direct detection of the biotin-containing transcripts using, e.g., Streptavidin-Alexa647 conjugate, and scanned utilizing conventional scanning methods. Image intensities of each spot on the array are proportional to the abundance of the corresponding biomarker in the subject sample.

The use of the array has one or more advantages for mRNA expression detection. First, the global expression of several to thousands of genes can be identified in a single sample at one time. Second, through careful design of the oligonucleotide probes, the expression of both mature and precursor molecules can be identified. Third, in comparison with Northern blot analysis, the chip requires a small amount of RNA.

The RBM3 mRNA may for example be extracted from formalin-fixed, paraffin-embedded tumor tissue. Accordingly, the sample of the methods of the present disclosure may be formalin-fixed and/or paraffin-embedded tumor tissue independent of if RBM3 protein or RBM3 mRNA is detected.

The inventors have realized that RBM3 mRNA analysis of the present disclosure may be incorporated in an mRNA-based assay designed to support individualized treatment planning. Such an assay may employ RT-PCR to analyze the expression of several genes.

As a fourth aspect of the present disclosure, there is provided a use of an RBM3 protein or RBM3 mRNA molecule as a marker of response to gemcitabine treatment of a mammalian subject having a cancer, wherein the protein or mRNA molecule is provided in a sample derived from the cancer of the subject.

Various examples of suitable types of samples for the fourth aspect are discussed above in connection with the method aspects.

The use of the fourth aspect may be entirely *ex vivo,* e.g., on previously obtained samples.

It is to be understood that the presence of the marker of the fourth aspect, or a relatively high level thereof, is indicative of a relatively high responsiveness to gemcitabine as compared to a relatively low responsiveness indicated by the absence or a relatively low level of the marker of the fourth aspect.

As a first configuration of a fifth aspect of the present disclosure, there is provided a use of an affinity ligand capable of selective interaction with an RBM3 protein as a treatment predictive agent for gemcitabine treatment of a mammalian subject having a cancer.

As a second configuration of the fifth aspect of the present disclosure, there is provided a use of an affinity ligand capable of selective interaction with an RBM3 protein for predicting the response to a gemcitabine treatment of a mammalian subject having a cancer.

As a third configuration of the fifth aspect of the present disclosure, there is provided a use of an affinity ligand capable of selective interaction with an RBM3 protein for indicating whether a mammalian subject having a cancer should be given a gemcitabine.

Various examples of suitable affinity ligands for the fifth aspect are discussed above in connection with the method aspects.

The use of the fifth aspect may be entirely *ex vivo.*

As a sixth aspect of the present disclosure, there is provided a product, namely gemcitabine, for use in a method of treatment of a mammalian subject having a cancer, wherein said method comprises the step of determining that an amount of RBM3 protein or RBM3 mRNA in a sample derived from the cancer of the subject is higher than a relevant reference value.

The sixth aspect thus relates to gemcitabine treatment of RBM3 high subjects. Various ways of determining that an amount of RBM3 in a sample derived from the cancer of the subject is higher than a relevant reference value are described above. For example, relevant reference values are discussed thoroughly.

Various embodiments of aspects four to six, in particular with regard to the type of cancer, are discussed above in connection with the method aspects.

In general, the inventors believe that the RBM3 protein expression is more relevant than the RBM3 mRNA expression in the context of the present disclosure.

### Examples

### Adenocarcinoma cohort analysis

### a) Material and methods

Archival formalin-fixed paraffin-embedded tissues from 175 patients, surgically treated with pancreaticoduodenectomy for primary pancreatic and periampullary adenocarcinoma, were collected from the Pathology departments at Lund and Malmö University Hospitals, Sweden. Patients were treated between January 1^{st} 2001 and December 31^{st} 2011. Ethical permission was obtained from the Local Ethics Committee of Lund University.

The median age of patients was 67 (38-83) years. Grade was recorded for all patients: 12 tumors were well differentiated, 62 were moderately differentiated, 97 were poorly differentiated, and 4 were undifferentiated. The heterogeneous cohort included 14 tumors originating from the duodenum, 51 tumors of papilla-ampulla intestinal type, 19 tumors of papilla-ampulla pancreatobiliary type, 45 distal bile duct tumors, and 45 pancreatic adenocarcinomas. Follow-up started at the date of surgery and ended at death or at December 31 st 2013, whichever came first. Treatment data was available for all patients. The majority of patients (n = 98) received no adjuvant treatment, 13 patients were treated with 5-FU only, 52 patients were treated with gemcitabine only, 5 patients were treated with oxaliplatin, 4 patients were treated with a combination of gemcitabine and capecitabine, and 3 patients were treated with a combination of gemcitabine and oxaliplatin.

All 175 cases were histopathologically re-evaluated on slides stained with hematoxylin and eosin. TMA:s were then constructed by sampling 3 x 1.0 mm cores from each of the 175 primary tumors.

For immunohistochemical analysis of RBM3 protein expression, 4 µm TMA-sections were automatically pretreated using the PT Link system and then stained in an Autostainer Plus (DAKO; Glostrup, Denmark) using the monoclonal anti-RBM3 antibody AAb030038 (Atlas Antibodies AB, Stockholm, Sweden).

The immunohistochemical staining was evaluated by two independent observers who were blinded to clinical and outcome data. Scoring differences were discussed to reach consensus. Immunohistochemical analysis of RBM3 expression with anti-RBM3 could be performed on 171/175 tumor samples. For assessment of nuclear RBM3 expression, the estimated fraction, 0.0-1.0 (1=100%), of cells with nuclear RBM3 expression was recorded for each core, as well as the predominant nuclear intensity as 0 (negative), 1 (weak), 2 (moderate) and 3 (strong). Fraction and intensity for each core was multiplied and a mean value of the cores was then calculated and used in the statistical analyses.

For further statistical analyses of RBM3 nuclear expression, these variables were dichotomized into high and low expression with the median value as cut-off. Five patients were excluded from survival analyses: Two who had received neoadjuvant chemotherapy, two who died within one month from surgery due to complications, and one who emigrated 5 months after surgery. Recurrence free survival (RFS) was defined from the date of surgery to the date of locoregional or distant recurrence. Kaplan Meier and the log rank test were applied to estimate differences in 5-year overall survival (OS) and RFS in strata according to high and low RBM3 expression. Hazard ratios (HR) for death and recurrence within 5 years were calculated by Cox regression proportional hazard's modelling in both unadjusted analysis and in a multivariable model adjusted for age, sex, Tstage, N-stage, differentiation grade, lymphatic invasion, vascular invasion, perineural invasion, infiltration in peripancreatic fat, resection margins, tumour location and adjuvant chemotherapy. A backward conditional method was used for variable selection in the adjusted model. All tests were two sided. P-values <0.05 were considered significant. All statistical analyses were performed using IBM SPSS Statistics version 22.0 (SPSS Inc., Chicago, IL,USA).

### b) Results

Initial analysis of the cohort revealed that 5-year overall survival (OS) and recurrence free survival (RFS) for the full cohort were both approximately 28%, as seen in Figure 1A and 1B, respectively.

As demonstrated in Figure 2, using the median RBM3 expression value as cut-off, Kaplan Meier analysis did not reveal any significant prognostic value for RBM3 expression in the full cohort, neither regarding OS nor RFS. The lack of association between RBM3 expression and prognosis in the entire cohort was confirmed in Cox regression analysis.

Surprisingly, when stratifying patients into subgroups according to levels of RBM3 expression, it was discovered that while patients with RBM3-low tumors actually had a significantly poorer survival if receiving adjuvant gemcitabine treatment, the patients with RBM3-high tumors receiving gemcitabine had a significantly improved survival compared to those patients not receiving gemcitabine treatment (Figure 3). The same results were obtained independent of whether or not the patients receiving other adjuvant chemotherapy were included in the analysis, (compare Figure 3 and Figure 4).

Also when analyzing the pancreatobiliary subgroup separately, patients with tumors expressing high levels of RBM3 had a significantly improved survival if they received adjuvant gemcitabine treatment, whereas patients with tumors expressing low levels of RBM3 had a significantly poorer survival if receiving gemcitabine treatment (Figure 5). Similar results were obtained also when analyzing RFS (Figure 6). For the intestinal subgroup there was a similar trend toward improved survival for RBM3-high patients receiving gemcitabine treatment, however, the number of patients in this subgroup was too small to obtain significant results (Figure 7B). For the intestinal subgroup, there was a similar survival rate for RBM3-low patients irrespective of gemcitabine treatment (Figure 7A).

When isolating the subgroup of patients with pancreatic adenocarcinomas, it could be shown that also in this subgroup, patients with tumors expressing high levels of RBM3 had a significantly improved survival if they received adjuvant gemcitabine treatment (Figure 9B). For the pancreatic adenocarcinoma patients with tumors expressing low levels of RBM3, there was a trend towards a poorer survival rate for te patients treated with gemcitabine (Figure 9A).

As shown in Figure 8, different cut-offs yielded similar results, which means that different reference values may be used for predicting the response to gemcitabine.

As demonstrated in the Table, there was a significant interaction between RBM3 expression and adjuvant gemcitabine treatment with regard to both OS (p = 0.002) and RFS (p = 0.002).

In conclusion, these results demonstrate that high expression of RBM3 in the primary tumor is a strong significant predictor of improved response to adjuvant gemcitabine treatment. Further, it is demonstrated that for a subgroup of cancer subjects, i.e. in patients with RBM3 low tumors, there is no benefit of gemcitabine. Instead, the survival in this subgroup is lower/shorter for the subjects given gemcitabine than for the subjects that have not been given the treatment.

**Table. Cox proportional hazards analysis of the impact of RBM3 median protein expression on survival and recurrence according to adjuvant gemcitabine treatment in the entire cohort and pancreatobiliary subgroup**

| | Death within 5 years | | | Recurrence | | |
|---|---|---|---|---|---|---|
| *Entire cohort* | HR (95%Cl) | *n (events)* | *p** | HR (95%Cl) | *n (events)* | *p** |
| No Gemcitabine | | | | | | |
| RBM3 low | 1.00 | 56 (34) | | 1.00 | 56 (31) | |
| RBM3 high | 1.72 (1.08-2.74) | 53 (39) | | 1.92 (1.20-3.07) | 53 (41) | |
| | *1.21 (0.71-2.06)* | | 0.002 | *1.35 (0.80-2.26)* | | 0.002 |
| Gemcitabine | | | | | | |
| RBM3 low | 1.00 | 27 (22) | | 1.00 | 27 (23) | |
| RBM3 high | 0.47 (0.25-0.90) | 30 (16) | | 0.58 (0.32-1.05) | 30 (21) | |
| | *0. 26 (0.11-0.63)* | | | *0.44 (0.23-0.83)* | | |
| *Pancreato-biliary type* | | | | | | |
| No Gemcitabine | | | | | | |
| RBM3 low | 1.00 | 25 (17) | | 1.00 | | |
| RBM3 high | 2.27 (1.23-4.18) | 29 (32) | | 2.57 (1.38-4.77) | 25 (16) | |
| | *1.81 (0.85-3.87)* | | <0.001 | *2.41 (1.08-5.37)* | 32 (31) | <0.001 |
| Gemcitabine | | | | | | |
| RBM3 low | | 21 (19) | | | | |
| RBM3 high | 0.36 (0.18-0.72) | 27 (16) | | 0.51 (0.27-0.97) | 21 (19) | |
| | *0.34* (*0*.*16*-*0*.*71*) | | | *0.50* (*0*.*25*-*0*.*99*) | 27 (21) | |

| | | | | | | |
|---|---|---|---|---|---|---|
| *p-value for term of interaction by Cox multivariable analysis including treatment, the binary covariate RBM3 expression, gemcitabine vs other/no treatment and a term of interaction. *Multivariable analysis adjusted for age, gender, tumour origin, tumour size, T-stage, N-stage, differentiation grade, involved margins, lymphatic growth, vascular growth, perineural growth and growth in peripancreatic fat.* | | | | | | |

### Establishment of a treatment prediction

### A non-limiting example

Following the establishment of a cancer in a patient, a tumor tissue sample from the patient is obtained. The tumor tissue sample may be obtained from a specimen from an earlier surgical removal of the tumor or from a tumor biopsy. Further, for the provision of a "negative reference", a sample is taken from archival material comprising tissue having low, or essentially lacking, RBM3 protein expression. Such archival tissue may for example be tissue having a pre-established low RBM3 protein expression level from the same cancer type as the one of the tested subject. Further, for the provision of a "positive reference", a sample is taken from archival tissue material of the same cancer type as the one of the tested subject, which archival material comprises tissue having high RBM3 protein expression.

The sample and reference material is fixated in buffered formalin and histo-processed in order to obtain thin sections (4 µm) of the sample material.

For immunohistochemical analysis of the RBM3 protein expression, the 4 µm TMA-sections are automatically pretreated using the PT Link system and then stained in an Autostainer Plus (DAKO; Glostrup, Denmark) using a selective anti-RBM3 antibody, such as the monoclonal antibody AAb030038 (Atlas Antibodies AB, Stockholm, Sweden).

Optionally, two control cell-lines may be used as a tool to validate the staining procedure; e.g. one slide with cells expressing RBM3 protein (positive cell line) and one slide having cells with indistinct weak or no RBM3 protein expression (negative cell line). The skilled artisan understands how to provide such cell lines, for example guided by the disclosure of Rhodes et al. (2006) The biomedical scientist, p 515-520. The control-line slides may be simultaneously stained in the same procedure as the other slides, i.e. incubated with the same primary and secondary antibodies.

If the control cell-lines are used, these are inspected to validate the staining procedure. If the cell-lines display staining results outside acceptable criteria, e.g. staining artifacts recognized by the skilled artisan, the staining of the tissue samples is considered invalid and the whole staining procedure is repeated with new slides. If the positive and negative cell-lines display strong staining intensity and indistinct weak or no staining intensity, respectively, the staining is considered as valid.

The immunohistochemical staining result is then evaluated by two independent observers who are blinded to clinical and outcome data. Scoring differences are preferably discussed to reach consensus.

For assessment of nuclear RBM3 expression, the estimated fraction, 0.0-1.0 (1=100%), of cells with nuclear RBM3 expression is recorded for each core. Further, the nuclear intensity for each core is recorded as 0 (negative), 1 (weak), 2 (moderate) and 3 (strong). The fraction and the intensity for each core are then multiplied and a mean value (staining score) of the cores is then calculated and used in the statistical analyses. The staining score will thus be in in the range of 0-3.

The observers performing the evaluation and determination are aided by visual inspection of the stained positive and negative reference slides.

The staining score is then compared to a reference value. As can be seen in the figures, a suitable reference value may be a staining score of 1.77, in particular if AAb030038 is used.

If the sample value/staining score is higher than 1.77, the physician responsible for the treatment of the patient concludes that the patient's responsiveness to gemcitabine is relatively high and optionally, that the patient should be given gemcitabine.

If however the sample value/staining score is lower than or equal to 1.77, the physician concludes that the patient's responsiveness to gemcitabine is relatively low and, optionally, to refrain from gemcitabine treatment. In the latter case, an alternative treatment may be applied instead.

## Claims

1. Method of determining whether a mammalian subject having a cancer belongs to a first or a second group, wherein subjects of the first group are more responsive to gemcitabine than subjects of the second group, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is higher than said reference value,
c1) concluding that said subject belongs to said first group; and
if said sample value is lower than or equal to said reference value,
c2) concluding that said subject belongs to said second group.

2. Method for determining whether a mammalian subject having a cancer is not in need of gemcitabine treatment, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is lower than or equal to said reference value,
c) concluding that the subject is not in need of gemcitabine treatment.

3. Non-treatment strategy method for a mammalian subject having a cancer, comprising the steps of:
a) evaluating the amount of RBM3 protein or RBM3 mRNA present in at least part of a sample derived from the cancer of said subject, and determining a sample value corresponding to said amount;
b) comparing the sample value obtained in step a) with a reference value; and,
if said sample value is lower than or equal to said reference value,
c) refraining from treating the subject with gemcitabine.

4. Method according to any one of the preceding claims, wherein said cancer is selected from the group consisting of non-small cell lung cancer, pancreatic cancer, urothelial cancer, breast cancer, esophageal cancer, lymphomas, pancreatic and periampullary cancers.

5. Method according to any one of the preceding claims, wherein said cancer is selected from the group consisting of duodenal cancer, ampullary cancer, distal bile duct cancer and pancreatic cancer.

6. Method according to any one of the preceding claims, wherein said cancer is selected from the group consisting of distal bile duct cancer, pancreatic cancer and ampullary cancer of the pancreatobiliary type.

7. Method according to any one of the preceding claims, wherein said sample is a tumor tissue sample.

8. Method according to any one of the preceding claims, wherein step a) comprises:
al) applying to said sample a quantifiable affinity ligand capable of selective interaction with the RBM3 protein to be evaluated, said application being performed under conditions that enable binding of the affinity ligand to RBM3 protein present in the sample; and
all) quantifying the affinity ligand bound to said sample to evaluate said amount.

9. Method according to claim 8, wherein the antibody fragments and derivatives are selected from the group consisting of Fab fragments, Fv fragments and single chain Fv (scFv) fragments.

10. Use *ex vivo* of an RBM3 protein or RBM3 mRNA molecule as a marker of response to gemcitabine treatment of a mammalian subject having a cancer, wherein the protein or mRNA molecule is provided in a sample derived from the cancer of the subject.

11. Use *ex vivo* of an affinity ligand capable of selective interaction with an RBM3 protein for predicting the response to a gemcitabine treatment of a mammalian subject having a cancer.

12. Use according to claim 11, wherein said affinity ligand is capable of selective interaction with a peptide whose amino acid sequence consists of SEQ ID NO: 1.

13. Use according to claim 12, wherein said affinity ligand is capable of selective interaction with an RBM3 fragment which consists of 20 amino acid residues or less, such as 15 amino acid residues or less, and comprises a sequence selected from SEQ ID NO: 8, 16, 17, 22, 24, 25 and 26.

14. Gemcitabine for use in a method of treatment of a mammalian subject having a cancer, wherein said method comprises the step of determining that an amount of RBM3 protein or RBM3 mRNA in a sample derived from the cancer of the subject is higher than a relevant reference value.

15. Gemcitabine for use according to claim 14, wherein the cancer is selected from the group consisting of distal bile duct cancer, pancreatic cancer and ampullary cancer of the pancreatobiliary type.
